# EUROPEAN PATENT SPECIFICATION

(11) **EP 3 838 347 B1**
(45) Date of publication and mention of the grant of the patent: **11.06.2025**
(21) Application number: 19217629.5
(22) Date of filing: 18.12.2019
(51) Int. Cl.: A61K 31/7048, A61P 31/04

(54) **EVERNIMICIN FOR TREATING DISEASES LIKE NECROTIC ENTERITIS**
EVERNIMICIN ZUR BEHANDLUNG VON ERKRANKUNGEN WIE NEKROTISCHER ENTERITIS
EVERNIMICINE POUR TRAITER DES MALADIES TELLES QUE L'ENTÉRITE NÉCROTIQUE

(43) Date of publication of application: 23.06.2021
(73) Proprietor: Intervet International B.V., 5831 AN Boxmeer (NL)
(72) Inventor: ULLRICH, Joachim, D-55270 Schwabenheim (DE); WARRASS, Ralf, D-55270 Schwabenheim (DE); JIRJIS, Faris, 07940 Madison, New Jersey (US)
(74) Representative: Intervet International B.V.

(56) References cited:
- ERJIAN WANG ET AL: "In Vivo Activity and Pharmacokinetics of Ziracin (SCH27899), a New Long-Acting Everninomicin Antibiotic, in a Murine Model of Penicillin-Susceptible or Penicillin-Resistant Pneumococcal Pneumonia", ANTIMICROBIAL AGENTS AND CHEMOTHERAPY, 1 April 2000 (2000-04-01), United States, pages 1010 - 1018, XP055744504, Retrieved from the Internet <URL:https://www.ncbi.nlm.nih.gov/pmc/articles/PMC89806/pdf/ac001010.pdf> DOI: 10.1128/AAC.44.4.1010-1018.2000
- A K GANGTJLY: "Ziracin, a Novel Oligosaccharide Antibiotic", THE JOURNAL OF ANTIBIOTICS, vol. 53, no. 10, 1 October 2000 (2000-10-01), pages 1038 - 1044, XP055744663
- MARIE ANNE PARADIS ET AL: "Efficacy of avilamycin for the prevention of necrotic enteritis caused by a pathogenic strain of Clostridium perfringens in broiler chickens", AVIAN PATHOLOGY, vol. 45, no. 3, 3 May 2016 (2016-05-03), GB, pages 365 - 369, XP055744362, ISSN: 0307-9457, DOI: 10.1080/03079457.2016.1165793
- W. E. SANDERS ET AL: "Microbiological Characterization of Everninomicins B and D", ANTIMICROBIAL AGENTS AND CHEMOTHERAPY, vol. 6, no. 3, 1 September 1974 (1974-09-01), US, pages 232 - 238, XP055744490, ISSN: 0066-4804, DOI: 10.1128/AAC.6.3.232

## Description

### Field of the invention

The present invention is directed to a composition comprising evernimicin for use in the treatment or prevention of a disease, such as necrotic enteritis, in animals.

### Background of the invention

Necrotic enteritis (NE) is one of the most important enteric diseases in poultry and is a high cost to the industry worldwide. It is caused by avian-specific, Necrotic Enteritis Beta toxin (NetB)-producing, strains of *Clostridium perfringens* that also possess in common other virulence-associated genes.

Necrotic enteritis (NE) in chickens is an enteric disease caused by C. *perfringens*, *a* Gram-positive anaerobic spore-forming, rod-shaped bacterium. According to the current classification, C. *perfringens* has five toxinogenic types (A, B, C, D, E), which are differentiated according to the production of four different major toxins (Alpha, Beta, Epsilon, Iota). The discovery in recent years of new toxins (Beta2, NetB, TpeL) in C. *perfringens* shows the need for an enhanced classification scheme. NE is caused by type A isolates and rarely by type C isolates. The recently discovered new toxin, NetB, is crucial for development of the disease. The discovery of the crucial role of the pore-forming toxin NetB led to the subsequent characterization of three pathogenicity loci (PAL) that are characteristic of NE isolates.

The intestinal number of C. *perfringens* in healthy and in NE-affected birds are different. The C. *perfringens* population is found to be normally less than l0² to 10⁴ colony-forming units (CFU) per g of the intestinal contents in the small intestine of healthy chickens compared to 10⁷ - 10⁹ CFU/g in diseased birds.

NE typically occurs in broilers aged between two and six weeks. Mortality can reach 1% per day with a total mortality of 10-40%. Clinical signs include depression, dehydration, diarrhea, ruffled feathers and lower feed intake. The gross lesions of the small intestine range from thin and friable walls to frank and extensive necrotic lesions. Two forms of the disease are described, clinical and subclinical. The clinical form appears with the clinical signs and mortality noted above. The subclinical form presents as poor performance (reduced growth, reduced feed efficiency) without mortality. This form of the disease can be diagnosed by reduced feed conversion, by gross lesions in the small intestine and by bacteriology. Most of the economic losses due to NE are related to the subclinical form.

The *netB* gene is mostly found in isolates from NE outbreaks, and is relatively uncommon in isolates from healthy birds.

The birds are infected via contaminated feed and litter containing C. *perfringens* spores and vegetative cells and the birds develop signs of necrotic enteritis usually at an early age. In addition to colonization and the cell damage caused by clostridial toxins like NetB, additional factors are needed to precipitate disease. Besides mucosal injury induced by co-infection with protozoa like *Eimeria ssp.*, viral infections or mycotoxins, also dietary factors like high protein diets, whole wheat or physiological factors like gut stasis are amongst the many predisposing factors associated with necrotic enteritis.

Treatment or prevention of necrotic enteritis is achieved by administering antibiotics. Inteprity^{®} is an in-feed composition comprising the antibiotic avilamycin for the prevention of necrotic enteritis in broiler chicken. Inteprity^{®}, however, has the drawback that it is not used as an in-water formulation and is also not used for the treatment of necrotic enteritis, but only for the prevention of necrotic enteritis.

BMD^{®}50 is a composition comprising the antibiotic bacitracin and BMD^{®}50 is available as an in-feed or an in-water formulation for the prevention or control of necrotic enteritis in several animals, such as poultry, such as broiler and replacement chickens, turkeys, quail, and in swine.

The treatment or prevention of necrotic enteritis with established antibiotics has several disadvantages.

Resistance against these established antibiotics are emerging so that antibiotics with specific activity against pathogenic *CI. perfingens* with high in-vivo activity are needed.

In particular, it is advantageous to develop new antibiotics for animals that are not used for the treatment of humans so that resistances of bacteria in humans cannot emerge. Thus, there is a need for new, alternative antibiotics not belonging to the critical antibiotic classes used in human health.

Additionally, some known antibiotics, such as avilamycin are not used for the treatment of necrotic enteritis and no in-water formulation of avilamycin exists.

Marie Anne Paradis et al (AVIAN PATHOLOGY; 45(3); 365-369) disclose the efficacy of avilamycin for the prevention of necrotic enteritis caused by a pathogenic strain of Clostridium perfringens in broiler chickens. Therefore, it is an object of the present invention to provide a composition for use in the treatment or prevention of a disease, e.g. necrotic enteritis, as well as a dosing regimen that overcome one or more of these problems.

Furthermore, it was an object to provide a composition that can be administered in-water and/or has less side-effects and/or has an improved efficacy, such as reduced mortality, an improved weight gain, an improved feed conversion ratio, a low intestinal lesion score, and/or a lower concentration required for effective treatment or prevention of a disease.

### Summary of the invention

One or more of these objects have surprisingly been solved by the use of evernimicin as described in more details below for use in the treatment or prevention of a disease, such as necrotic enteritis in animals, especially chickens.

The current invention is directed to Evernimicin of the formula 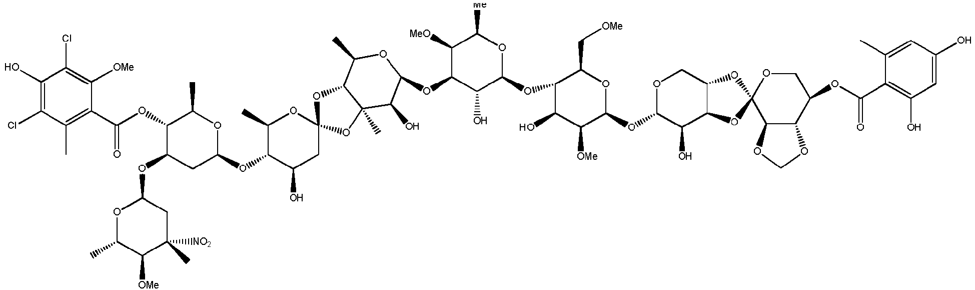 for use in the treatment or prevention of necrotic enteritis in poultry Evernimicin is an oligosaccharide orthosomycin antibiotic which binds to the large ribosomal subunit 50S. Evernimicin can be obtained by fermentation from *Micromonospora carbonaceae* (see i.a. Chu et al. J. Nat. Prod. 2002, 65, 1588-1593 or Wagman et al. Antimicrobial agents and chemotherapy 1964, 10, 33-37).

Manufacturing processses to obtain evernimicin have been described in prior art. It has surprisingly been found that evernimicin is not only highly active against pathogenic C. *perfringens* in vitro, but also exhibits excellent properties in vivo for the treatment or prevention of necrotic enteritis as well as mortality associated with said disease.

It has further been found that evernimicin is more active in vivo and leads to fewer mortalities, a higher weight gain and a lower feed conversion rate (considered effective) compared to the standard antibiotic bacitracin used for the treatment or prevention of necrotic enteritis in chickens.

It was also found that evernimicin can be administered via drinking water.

The present invention also relates to a kit of parts comprising the composition according to the present invention and instructions of administration.

### Detailed description

The following definitions are relevant in connection with the embodiments of the present invention.

### Definitions

"Growth" and "enhancing growth" are terms generally known in the art and refer to increases in either, or both, weight and size (e.g., height, width, diameter, circumference, etc.) over that which would otherwise occur without implementation of the methods and/or administration of the compositions of the present invention. Growth can refer to an increase in the mass (e.g., weight or size) of the entire animal or of a particular tissue (e.g., muscle tissue in general or a specific muscle). Alternatively, growth can indicate a relative increase in the mass of one tissue in relation to another, in particular, an increase in muscle tissue relative to other tissues (e.g., adipose tissue).

"Feed conversion ratio" is defined as the ratio of feed mass input to body mass output and has a significant impact on the economical profitability of animal rearing.

"Feed efficiency" refers to: feed consumed / total live body weight. "Mortality adjusted Feed efficiency" refers to feed consumed / (total live body weight + body weight of mortalities).

"Average daily gain" refers to body weight gain / number of days.

"ppm" is an abbreviation of parts per million. ppm is a value that represents the part of a whole number in units of 1/1000000. "ppm' is dimensionless quantity, a ratio of 2 quantities of the same unit. For example, a 5 ppm concentration of zilpaterol means 5 mg of zilpaterol per 1 kg of feed.

"Poultry" are domesticated birds kept by humans for the purpose of producing eggs, meat, and/or breeding stock. These most typically are members of the superorder Galloanserae (fowl), especially the order Galliformes (which includes chickens, quails and turkeys) and the family Anatidae (in order Anseriformes), commonly known as "waterfowl" (e.g. domestic ducks and domestic geese). Poultry also includes other birds which are killed for their meat, such as pigeons or doves or birds considered to be game, like pheasants. Chickens (Gallus gallusdomesticus) are domesticated fowl.

"Broiler" or "Broiler chickens" are chickens raised for meat production. In most countries the broiler chickens are raised in big operations, housing thousands of animals on an industrial scale.

"About" means a range around the number. When used in connection with numbers such as concentrations, doses, dose rates and weights, about means plus or minus 25%.

"Feed" means edible materials which are consumed by animals and contribute energy and/or nutrients to the animals' diet (American Association of Feed Control Officials, 5th Ed. 2014, p 178).

"Medicated feed" is any feed which contains drug ingredients intended or presented for the cure, mitigation, treatment, or prevention of disease of animals other than man or which contains drug ingredients intended to affect the structure or any function of the body of animals other than man (American Association of Feed Control Officials, 5th Ed., p 187).

The following types of Medicated Products are defined in US 21 CFR § 558.3:
"Type A Medicated Article": (also called Premix) is intended solely for use in the manufacture of another Type A Medicated Article or a Type B or Type C Medicated Feed. It consists of an animal drug (s) with or without a carrier (e.g., calcium carbonate, rice hull, corn, gluten) with or without inactive ingredients.

"Type B Medicated Feed": (Type B feed) (previously called Concentrate) is intended solely for the manufacture of other medicated feeds (Type B or Type C). It contains a substantial quantity of nutrients including vitamins and/or minerals and/or other nutritional ingredients in an amount not less than 25 percent of the weight. It is manufactured by diluting a Type A Medicated Article or another Type B Medicated Feed.

"Type C Medicated Feed": (Type C feed) is intended as the complete feed for the animal or may be fed "top dressed" (added on top of usual ration) on or offered "free-choice" (e.g., supplement) in conjunction with other animal feed. It contains a substantial quantity of nutrients including vitamins, minerals, and/or other nutritional ingredients. It is manufactured by diluting a Type A Medicated Article or a Type B Medicated Feed. A Type C Medicated Feed may be further diluted to produce another Type C Medicated Feed.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the composition specified by "consisting of".

The term "weight-% "is to be understood to refer to the weight amount of the component relative to the total weight amount of feed or drink, if not specified otherwise.

Other definitions for selected terms used herein will be found within the description of the invention and apply throughout. Unless otherwise defined, all other scientific and technical terms used herein have the same meaning as commonly understood by individuals who are skilled in the art.

It is to be understood that the weight of evernimicin is based on the free form of evernimicin (in contrast to being based on the weight of e.g. a pharmaceutically acceptable salt).

As used herein, the term evernimicin relates to evernimicin as well as pharmaceutically acceptable salts, solvates, hydrates, prodrugs, derivatives, stereoisomers and mixtures thereof.

Evernimicin comprises ziracin (SCH 27899) CAS 53024-98-9 of the following formula:

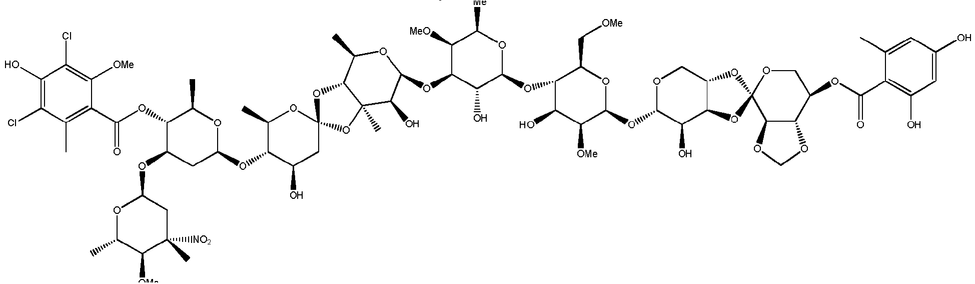

The term "pharmaceutically acceptable salt" refers to salts prepared from pharmaceutically acceptable non-toxic bases or acids including inorganic or organic bases and inorganic or organic acids.

The term "prodrug" as used herein means a derivative of a compound described herein that can hydrolyze, oxidize, or otherwise react under biological conditions (in vitro or in vivo) to provide a compound of the invention. Prodrugs may only become active upon such reaction under biological conditions, or they may have activity in their unreacted forms.

The term "derivative" refers to compounds having the structure of evernimicin but containing additional substituents and/or functional groups.

Evernimicin may exist in both unsolvated and solvated forms. The term "solvate" is used herein to describe a molecular association comprising one or more pharmaceutically acceptable solvent molecules, e.g. water or ethanol. The term "hydrate" is used when said solvent is water.

Preferred embodiments according to the invention are defined hereinafter. The preferred embodiments are preferred alone or in combination. Further, it is to be understood that the following preferred embodiments refer to all aspects of the present invention, i.e. the composition for use and the kit of parts.

In an embodiment, evernimicin, or a composition comprising evernimicin is used for the treatment and/or prevention of a disease. The disease may be any disease associated with and/or caused by bacteria belonging to *Clostridium* spp., such as C. *perfringens, C. difficile, C. chauvoei* and C *novyi.*

Accordingly, evernimicin may be used for the treatment or prevention of any one of the following diseases: C. *chauvoei* causes blackleg in cattle and sheep. C. *novyi* induces infectious necrotic hepatitis (black disease) in sheep and occasionally cattle. C. *difficile* causes antibiotic-associated colitis in humans and diarrhea and colitis in horses, neonatal swine, calves and other animals. C. *perfringens* is an important cause of food poisoning and gas gangrene in humans and of a variety of enteric diseases in various animal species, like necrotic enteritis in birds, diarrhea in pigs, colitis in horses and enterotoxaemia in cattle, sheep and goats.

In an embodiment, evernimicin is used for the treatment or prevention of necrotic enteritis in an animal. In another embodiment, evernimicin is used for the treatment or prevention of necrotic enteritis in an animal caused by C. *perfringens.*

In one embodiment evernimicin is used to prevent, control or treat necrotic enteritis caused or complicated by *CI. perfingens.*

In another embodiment evernimicin is used as an aid in prevention of necrotic enteritis.

For prevention evernimicin is administered to a flock of chicks that is at risk of developing necrotic enteritis but none of the animals of the flock yet showing clinical symptoms of necrotic enteritis associated with *CI. perfringens.*

In one embodiment evernimicin is used to treat necrotic enteritis in a flock of chickens, this means starting the medication with evernimicin at the first appearance of clinical signs, as described earlier in at least one animal in the flock.

In an embodiment, evernimicin is used for the treatment or prevention of necrotic enteritis in an animal, wherein the animal is poultry. In another embodiment, the animal belongs to the superorder *Galloanserae.* In a preferred embodiment, the animal is chicken or turkey. In an even more preferred embodiment, the animal is chicken, most preferably broiler chicken, replacement chicken, laying hen, especially broiler chicken.

A group of chickens is a group, brood or a flock, which is the general term used to describe a group of animals, especially poultry that are assembled or herded together. These flocks are in commercial husbandry kept together in a confined area, usually a chicken house or stable. Broiler houses are for intensive chicken farming, where the birds are kept inside the poultry house permanently.

In such confined area the contact to other flocks of animals of the same species, are generally avoided so that a separate group of animals is in contact with each other. This is important for the spreading of a contagious disease such as necrotic enteritis that can be transferred from one animal to another either directly or indirectly via the litter on the floor of the poultry house.

It is important that these flocks of animals are treated at the same time to reach the same protection status for all animals in such a flock, or in case of treatment, treat all animals that are either infected, or at last at risk of infection with pathogenic agents involved with necrotic enteritis at the same time.

It is even more preferred to treat all animal flocks on the farm at the same time to prevent spreading of the disease.

It is therefore very important that such large groups of animals on commercial scale (flocks or herds) that can include several thousands of chickens can be reached at the same time via feed medication or medicated drinking water.

In such situation preferably evernimicin is administered continuously for approximately one week, or 5 to 7 days, or alternatively as long as the clinical signs of necrotic enteritis is at least one animal of the flock persist. In one embodiment, the duration of evernimycin administration (evernimycin administration period) is 7 daysln such situation in a preferred embodiment evernimicin is administered via drinking water. In another embodiment evernimicin is continously fed for 21 days in feed or drinking water..The main purpose of such administration is the prevention of mortalities in the treated flock and the prevention of performance loss, i.e. a reduced weight gain and increased feed conversion ratio.

In an embodiment, evernimicin is administered orally. Oral administration may take place in feed or in water, such as drinking water that are useful to reach all animals in the flock to be treated at the same time.

Evernimycin can be administered every day (daily) to the chicken, especially the whole flock during an evernimicin administration period.. Administration in the context of the invention includes the availability of evernimycin in the feed or drinking water of the chicken either *ad libitum* or in the form of batch feeding. Such a daily administration includes continuous administration i.e. during the administration period evernimycin is included in all feed (or drinking water) that is offered to the broiler chicken for consumption or evernimycin is included in at least one feed or feeding component that is fed to the chicken continuously during the whole administration period. The period of time that the evernimycin is administered to the chicken is called the evernimycin administration period.

In further embodiments, the administration period begins on about 21 days of age, or begins on about 28 days of age, or begins on about 35 days of age or begins on about 42 days of age or begins on about 49 days of age.

Of importance is that evernimicin is administered when the feed to the animal contain proteins (or essential amino acids, e.g. lysine) and energy in the appropriate proportion, the presence of which in feed is a well-known prerequisite for muscle growth in all species. The dietary protein and energy requirements for various species are well known for those skilled in the art. As an example, a maize-soybean meal diet can be used for broiler chicken, wherein the crude protein and energy concentration should preferably not be less than NRC requirements or current industry practices.

However, increased growth rate may be further increased by feeding a higher dietary crude protein concentration and/or including certain amino acids that are known to be rate-limiting (e.g., lysine) for growth. In another embodiment, the methods of the invention further comprise administration of greater than normal amounts of protein or essential amino acids. Especially in such a situation the administration of evernimicin is very beneficial to prevent or control necrotic enteritis in the animals.

For distribution purposes, an admixture of a high concentration of evernimycin can be prepared in a suitable premix (e.g., Type A Medicated Article) to be included in the feed. The premix is distributed to the end users in bags containing said premix. The end-user, usually the farmer/commercial producer raising poultry, will further dilute the premix into the regular feed that is used for said poultry.

Said premix may contain evernimycin in diluents. The premix promotes a uniform distribution of the active ingredients in the finished feed into which the premix is blended. It thus performs an important function by ensuring proper distribution of the active ingredient throughout the feed offered to poultry.

As diluents a variety of common solid carriers may be used (e.g., cereal by-products, such as wheat flour, wheat bran or de-oiled rice bran, but also corn meal, alfalfa meal , calcium carbonate, soybean meal, cottonseed oil meal, linseed oil meal, sodium chloride, cane molasses, bone meal, corncob meal, rice kernel, and the like).

If desired, such concentrates or premixes may contain other ingredients, such as minerals, trace elements, or vitamins.

Such concentrates or premixes are then added to the feed of the animal in an amount sufficient to provide the desired concentration in the resulting feed mixture and to provide the desired dosage amounts in the range previously described by suitably mixing with the feed. The known methods of formulating, mixing, and processing feeds which are normally used in the animal feed arts are entirely appropriate for manufacturing feeds containing evernimycin.

Poultry feed, especially chicken broiler feed, for use in the method of the present invention contains evernimycinin a concentration of from about 1 ppm to about 45 ppm.

In an embodiment, evernimicin is used for treatment or prevention of intestinal lesions caused by or at least influenced by pathogenic C. *perfringens,* especially its toxins. Preferably, after treatment of evernimicin the intestinal lesion score is reduced by 10 to 100 % compared to untreated animals. In another preferred embodiment, the intestinal lesion score and/ or mortality is reduced by 10 to 30%, 30 to 50%, 50 to 70 %, 70 to 100%, 10 to 20%, 20 to 30%, 40 to 50%, 60 to 70%, 80 to 90% or 90 to 100% compared to untreated animals that have been challenged / are infected with pathogen *CI. perfringens.*

The term "intestinal lesion score" relates to the mean lesion scoring system according to Table 3 of the present description and is measured according to the methods of the description.

In an embodiment, the administration of evernimicin leads to an average weight gain increase of 5 to 100% compared to untreated animals. In a preferred embodiment, the average weight gain is increased by 5 to 20%, 20 to 30% or 30 to 40% compared to untreated animals that have been challenged / are infected with pathogen *CI. perfringens.* In a particularly preferred embodiment, the average weight gain is increased by 5 to 15% compared to such untreated animals. The average weight gain is determined according to the methods in the Examples, wherein the animal is challenged with C. *perfringens* on day 17 and the average weight gain is determined on day 35.

In another embodiment, the average mortality-adjusted feed conversion rate is decreased by 2 to 30% compared to untreated animals that have been challenged / are infected with pathogen *CI. perfringens.* In a preferred embodiment, the average mortality-adjusted feed conversion rate is decreased by 2 to 5%, 5 to 10%, 10 to 20% or 20 to 30%. In a more preferred embodiment, the average mortality-adjusted feed conversion rate is decreased by 2 to 5%. The average mortality adjusted feed conversion rate is determined according to the methods in the Examples, wherein the animal is challenged with C. *perfringens* on day 17 and the average mortality-adjusted feed conversion rate is determined on day 35.

In another embodiment, evernimicin is used in form of a composition comprising an excipient. In one preferred embodiment, the composition comprises a powder. The term "composition" refers to both, the sold pre-mix formulation for mixing with feed or drinking water as well as the final mixture of said pre-mix with feed (medicated feed) or drinking water (medicated drinking water), that is administered to the animal. If not stated otherwise, concentration values of evernimicin refer to the concentration of evernimicin in the mixture that is administered (and not the pre-mix formulation).

The feed premix for use in this invention can be a Type A Medicated Feed, a Type B Medicated Feed as defined in US 21 CFR § 558.3.

The feed premix can be used (by dilution) to prepare a Type A Medicated Feed, a Type B Medicated Feed or a Type C Medicated Feed as defined in US 21 CFR § 558.3.

In one embodiment, the composition comprises feed pellets or granules of the same type as the feed that is used for the animals.

In another embodiment evenimicin is administered to a flock of poultry animals (or pigs) via drinking water. Medication via drinking water systems is preferred to be routinely used for administering medicaments to intensively reared animals because of the easiness of administration to a number of animals at the same time.

Many swine and poultry farms are already equipped with the necessary devices to administer medication via drinking water systems. Such drinking water systems on farms are complex systems of tanks, pipes, coils, pen drinkers and nipples. An average stable may contain hundreds of meters of pipes with many coils and hundreds of individual cups and/or nipples.

Evenimicin can be delivered to the animals through a drinking water system of choice by means of mixing and diluting the composition with water in the central water tank or separate storage tank.

Alternatively, the composition is injected continuously into a high- or low- pressure ring system for drinking water distribution, using a dosage dispenser or dosing pump system or proportioner medication system.

Dosing pump systems rely on a pump that delivers measured amounts of a concentrate into the water pipes at a typical dilution of 1-5%. Within the dosing pump systems, electronic dosing pump systems such as KONTI-DOS from Buerkert or mechanical dosing pump such as DOSATRON^{®} water powered dosing pump, DOSMATIC^{®} water-driven, proportional medicators can be used. The variety of field installations also concerns the water supply systems themselves: dead end or closed loop systems in different lengths with different pipe materials (e.g. PVC, galvanized iron) and the drinkers which are adapted to the target animals such as bell drinkers, nipples.

Medicated drinking water is produced by mixing and diluting the amount (volume) of a composition with water until the concentration of evernimicin that provides an effective amount for the number of animals treated with a volume of drinking water that corresponds to the volume that will be consumed during the treatment period to a large extend by the animals.

A pre-diluted solution that can be used in the preparation of medicated drinking water can be prepared from a concentrated solution or a powder by mixing with a defined volume of drinking water.

Such a pre-diluted solution can be further diluted in 1 to 5 steps to manufacture medicated drinking water. Such pre-diluted solution comprises 1 to 85 % v/v of water.

In one embodiment the pharmaceutical composition can be used to treat or prevent necrotic enteritis of animals, especially livestock animals (e.g., cattle, poultry and pigs) with evernimicin via drinking water systems.

In an embodiment, the composition comprises a pH-modifier, preferably adapting the pH value within the range of from pH 6.5 to 9.0. In another embodiment, the pH modifier results in a pH value of from 6.5 to 8, or from 7 to 8.

It is to be understood that the pH value refers to the measurement in water, at an effective concentration of evernimicin. The pH modifier allows for the adjustment of the pH, which can lead to a more stable form of evernimicin and/or improve the dissolution of evernimicin in water and thus a more effective treatment.

In another embodiment, evernimicin is administered as an aqueous solution via the drinking water. The term "aqueous solution" as used herein refers to evernimicin in water, wherein substantially all of evernimicin is dissolved. Solutions can be either "ready- to- use" (concentrated) solutions, in which all evernimycin is dissolved in a liquid solvent or prepared by way of a "soluble powder" in which a solid powder is dissolved in a liquid. Alternatively, medicated drinking water can be prepared using dispersions of evernimicin in water, this means evernimicin is present as particulates in a liquid dispersion medium.

The concentration of evernimicin to be administered may vary depending on the reason underlying the administration. Prevention of necrotic enteritis can be achieved with a lower concentration of evernimicin relative to the concentration of evernimicin required for efficient treatment of necrotic enteritis.

In an embodiment, evernimicin is administered at a concentration of 1 to 500 mg/L, 1 to 400 mg/L, 1 to 300 mg/L, 1 to 200 mg/L or 1 to 100 mg/L. In a preferred embodiment, evernimicin is administered at a concentration of 10 to 500 mg/L, 10 to 400 mg/L, 10 to 200 mg/l or 10 to 100 mg/L. In another preferred embodiment, evernimicin is administer at a concentration of 40 to 500 mg/mL, 40 to 400 mg/L, 40 to 300 mg/L, 40 to 200 mg/L or 40 to 100 mg/L. These are typical concentration ranges for the treatment of a disease.

In another embodiment, evernimicin is administered as an aqueous solution and the concentration of evernimicin is 1 to 500 mg/L, preferably 10 to 200 mg/L, more preferably 40 to 100 mg/L. It is preferred that this administration is used for the treatment of a disease.

In an even more preferred embodiment, evernimicin is administered as an aqueous solution at a concentration of 40 to 100 mg/L for the treatment of necrotic enteritis in chicken.

In another embodiment, evernimicin is administered in feed containing 1 to 300 ppm, 1 to 150 ppm, 1 to 100 ppm 1 to 45 ppm, 1 to 30 ppm, 15 to 300 ppm, 15 to 150 ppm, 15 to 100 ppm or 15 to 45 ppm. In a preferred embodiment, evernimicin is administered in feed containing 1 to 100 ppm. In a more preferred embodiment, evernimicin is administered in feed containing 1 to 45 ppm. It is preferred that this administration is used for the prevention of a disease.

Evernimicin can be administered in-water and in-feed at the same time. It is, however, also possible to administer evernimicin first in water and then subsequently in feed or vice versa. Furthermore, the concentration of evernimicin in-water and in-feed can be the same but can also be different. Additionally, evernimicin can be administered for the treatment and for the prevention of a disease (such as necrotic enteritis) at the same time or at a different time (e.g. consecutively). In one embodiment, the administration of evernimicin for the treatment of a disease is followed by the administration of evernimicin for the prevention of a disease, to prevent any new infections after successful treatment.

The pre-mix compositions containing evernimicin may or may not be the same for in feed and in drink compositions.

In an embodiment, evernimicin is first administered for the treatment of necrotic enteritis at a first concentration and subsequently evernimicin is administered for the prevention of necrotic enteritis at a second concentration. In another embodiment, evernimicin is administered at the same time at a first concentration in water and at a second concentration in feed.

In a particularly preferred embodiment, evernimicin is first administered as aqueous solution at a concentration of 1 to 500 mg/L, preferably 10 to 200 mg/L, more preferably 40 to 100 mg/L, and at the same time or subsequently, evernimicin is administered in feed at a concentration of 1 to 150 ppm, 1 to 100 ppm or 1 to 45 ppm.

In an even more preferred embodiment, evernimicin is administered in-feed at a concentration of 1 to 45 ppm for the prevention of necrotic enteritis.

Broiler Starter should be given for ten days. Broiler Grower feed will normally be fed for 14 to 16 days. After that, Finisher feed is provided. Each of the broiler dets have an optimized compodition to optimally support growth of the animal. The transition from Starter feed to Grower feed will generally involve a change of texture from crumbs to pellets. Therefore each feed transition period is especially critical because the chickens intestinal tract needs to adapt to the structure and composition of the new feed. Evernimicin administration during such transition period can help to prevent growth of pathogenic bacteria causing NE, especially pathogenic *CI. perfringens.* This evernimicn administration can be performed in-feed or via drinking water, but especiaaly via drinking water.

In another embodiment, especially for prevention of necrotic enteritis evernimicin is administered consecutively for a duration of 1 to 49 days, 1 to 42 days, 1 to 35 days, 1 to 28 days, 1 to 21 days or 1 to 14 days. In another embodiment, evernimicin is administered for a duration of 7 to 49 days, 7 to 42 days, 7 to 35 days, 7 to 28 days, 7 to 21 days or 7 to 14 days. In yet another embodiment, evernimicin is administered for a duration of 14 to 49 days, 14 to 42 days, 14 to 35 days, 14 to 28 days, or 14 to 21 days. In another embodiment, evernimicin is administered for a duration of 21 to 49 days, 21 to 42 days, 21 to 35 days or 21 to 28 days.

In a preferred embodiment, evernimicin is administered for a duration of 1 to 14 days or 7 to 21 days. The duration of administration may vary for treatment and prevention, respectively. For the treatment of necrotic enteritis, evernimicin is administered for 1 to 21 days, preferably 1 to 14 days. For the prevention of necrotic enteritis, a prolonged, consecutive administration is favored and evernimicin is administered for a duration of 1 to 49 days, preferably for 7 to 49 days.

Alternatively, it may be favorable to administer evernimicin according to the weight of the animal. In one embodiment, evernimicin is administered in an amount of 10·x to 500·x ppm based on the total weight of feed/drink, wherein x corresponds to the weight of the animal in kg.

In one embodiment, evernimicin is administered orally as an aqueous solution via the drinking water at a concentration of evernimicin of 1 to 500 mg/L for 1 to 14 days for the treatment of necrotic enteritis.

In another embodiment, evernimicin is administered orally in feed containing 30 to 150 ppm of evernimicin for a duration of 1 to 49 days for the prevention of necrotic enteritis.

In an embodiment, evernimicin is administered as an aqueous solution via drinking water at a concentration of evernimicin of 1 to 500 mg/L and/or evernimicin is administered in-feed containing 1 to 150 ppm of evernimicin. In another embodiment, evernimicin is administered as an aqueous solution at a concentration of evernimicin of 40 to 100 mg/L and/or evernimicin is administered in-feed containing 1 to 45 ppm of evernimicin.

The compositions and methods of this invention may further include, in combination with evernimicin, one or more other active ingredients. Other active ingredients include any material which can be added to the feed or drinking water to enhance the animal's performance, health, and/or well-being. Examples of such include anticoccidials such as amprolium, clopidol, decoquinate, diclazuril, halofuginone hydrobromide, lasalocid, monensin, narasin, nicarbazin, robenidine, salinomycin, semduramycin, sulfadimethoxine / ormetoprime, zoalene, and feed additives (i.e., antibiotics and ionophores) used for improved performance such as, bacitracin, bambermycin, chlortetracycline, lincomycin, neomycin, oxytetracycline, penicillin, roxarsone, tylosin, and virginiamycin, enzymes, minerals, vitamins and other supplements. The above active ingredients are also used for various health related concerns. The person skilled in the art will recognize that the agents listed above are examples of a wide range of feed additives which may be used. Other examples are referred to in "2012 Feed Additive Compendium" and "Handbook of Feed Additives 2012".

### Examples

### Example 1: In vitro activity of evernimicin against Clostridium perfringens

The antimicrobial activity against *Clostridium perfringens* field isolates from diseased chickens from different areas was determined for the orthosomycins ziracin (in the following referred to as evernimicin) and avilamycin (Inteprity^{®}) and bacitracin (BMD^{®}50) in Table 1.

Minimal inhibitory concentrations (MIC) were determined according to Clinical and Laboratory Standard Institute (CLSI) guideline M11-A8 (CLSI. Methods for Antimicrobial Susceptibility Testing of Anaerobic Bacteria; Approved Standard-Eight Edition. CLSI document M11-A8 Wayne, PA: Clinical and Laboratory Standards Institute; 2012).

In short, 40 epidemiologically unrelated isolates of *Clostridium perfringens* from diseased chicken of different European countries, a C. *perfringens* type strain and a *Bacillus fragilis* reference strain were harvested from agar plate cultures and were dispensed at a density of ~ 5x10⁵ cfu/ml into 96-well plates containing brucella broth supplemented with vitamin K1, hemin and lysed horse blood. The 96-well plates contained evernimicin,avilamycin or bacitracin in two-fold dilutions from 64 µg/ml to 0.063 µg/ml. The plates were incubated anaerobically at 37 °C for ~ 40 hrs.

The lowest concentration of evernimicin or avilamycin at which no visible growth by the unaided eye occurred was recorded as the MIC. The MIC₉₀ denote the concentrations at which 90% of all tested strains do not show visible growth.

Good antimicrobial activity of evernimicin against 40 C. *perfringens* chicken field isolates was determined (Tab. 1). Unexpectedly, the antimicrobial activity of evernimicin was significantly better than that of avilamycin, the active component of the marketed product Inteprity^{®} for the prevention of necrotic enteritis in chicken and bacitracin, the active component of the market product BMD^{®}50 for the same disease The MIC₉₀ for evernimicin was 2 µg/ml and ≥ 64 µg/ml for avilamycin and bacitracin, respectively.

**Tab.1: MIC distribution of C. perfringens poultry isolates for 3 antimicrobials**

| | Number of *C. perfringens* strains (n=40) | | |
|---|---|---|---|
| MIC (µg/ml) | Evernimicin | Avilamycin | Bacitracin |
| ≤ 0.25 | 20 | 0 | 0 |
| 0.5 - 4.0 | 20 | 34 | 16 |
| 8.0 - 64.0 | 0 | 0 | 16 |
| > 64.0 | 0 | 6 | 8 |

### Example 2: In vivo activity of evernimicin in- feed

The ability of evernimicin to prevent an outbreak of necrotic enteritis in broiler chicken was demonstrated in an experimental infection model in which medication occurred prior to the infection of birds with C. *perfringens.*

The infection model is based on published protocols for the induction of necrotic enteritis in chicken (Shojadoost et al. 2012, Veterinary Research, vol. 43, p. 74 and Paradis et al. 2016, Avian Pathology, vol. 45, pp. 365-369).

In short, immediately after hatching broiler chicken were divided into 5 groups of 600 birds each. The birds of each group were distributed to 12 pens of 50 birds each. All birds received starter feed from days 0-13, grower feed from day 13-28 and finisher feed from day 28-35.

The study consisted of two control groups, two evernimicin-treated groups and one reference group receiving bacitracin (BMD^{®}50), a marketed product for the control and treatment of necrotic enteritis.

The groups are depicted in Tab. 2. The first control group did not receive medication nor challenge, whilst the second control group did not receive medication and was challenged on day 17 with a virulent C. *perfringens* chicken isolate.

Reference group 3 was fed feed containing bacitracin at 55 ppm, the label concentration for the control of necrotic enteritis. Evernimicin-treated groups were fed feed containing evernimicin at 45 ppm and 100 ppm.

Medicated feed was provided from day 0 to day 35 with the exception on day 17 when birds were challenged. On day 17 feed was removed in the morning and the birds were starved for 4-6 hrs.

Then birds in groups 2-5 received feed containing a virulent C. *perfringens* strain derived from an outbreak of necrotic enteritis in chicken. The challenge consisted of a 5 hr C. *perfringens* growth culture in thioglycolate medium containing 2-9 x 10⁸ cfu/ml. For challenge each bird received 2-3 ml C. *perfringens* culture mixed into approximately 25 g of feed.

The challenged feed was offered to the birds for 2-3 hrs. After challenge the empty trays were replaced by medicated grower feed for treatment groups 3-5 and unmedicated grower feed for the two control groups 1, 2.

At day 23 six animals per pen were randomly selected and gut lesions were scored according to the parameters laid out in Tab. 3.

The number of birds scored for lesions was decreased and replaced by the number of birds that died from necrotic enteritis prior to lesion scoring. Mortality after challenge day 17 with a minimum lesion score of 2 for dead or moribund animals was recorded as mortality associated with necrotic enteritis.

Feed consumption and pen weight were determined at the ends of the 3 feeding periods to assess average bird weight gain and the mortality-adjusted feed conversion rate. The averages of 12 pens/treatment group were calculated.

Calculation of average bird weight gain per pen: (pen weight on day 35/number of birds on day 35) - (pen weight on day 0/number of birds on day 0).

Calculation of mortality-adjusted feed conversion rate per pen: Feed consumed day 0-35 / ((day 35 pen weight - day 0 pen weight) + weight of dead and removed birds from day 0-35).

**Table 2: Treatment groups in experimental necrotic enteritis infection model**

| Treatment Group | Treatment | In feed concentration | Challenge |
|---|---|---|---|
| 1 | None | n.a.* | none |
| 2 | None | n.a. | *C. perfringens* |
| 3 | Bacitracin (BMD^{®}50) | 55 ppm** | |
| 4 | Evernimicin | 45 ppm | |
| 5 | Evernimicin | 100 ppm | |

| | | | |
|---|---|---|---|
| * not applicable ** parts per million | | | |

**Table 3: Lesion scoring system**

| Score | Description |
|---|---|
| 0 | Normal, no NE lesions |
| 1 | Small intestinal wall is thin and flaccid, thickened mucus |
| 2 | 1-6 necrotic enteritis pocks. Minor ulceration and necrosis |
| 3 | more than 6 pocks or coalescing of pocks |
| 4 | Extensive areas of necrosis and ulceration of the small intestinal membrane; significant hemorrhage, layer of fibrin and necrotic debris on mucus membrane |
| 5 | Dead or moribund and lesion score of 2 or more |

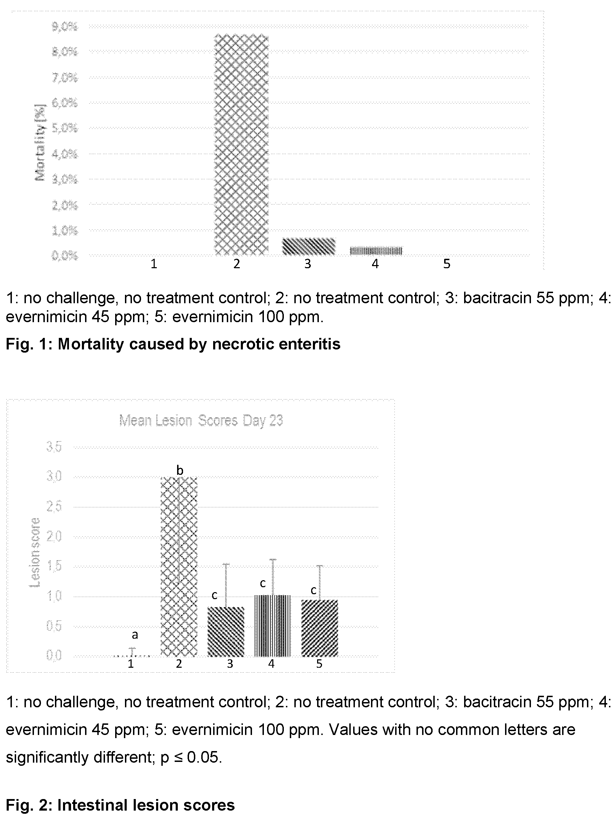

The assessment of mortality caused by necrotic enteritis (NE) (Fig. 1) clearly demonstrated high mortality in the challenged, untreated group 2 and no NE-induced mortality in the non-challenged group 1. Therefore, the model successfully induced the clinical manifestations of necrotic enteritis as observed in the field. Strong reduction of NE-induced mortality was observed in both evernimicin treated groups, clearly demonstrating the effectiveness of evernimicin in preventing necrotic enteritis. Intestinal lesion scores on day 23 (Fig. 2) revealed strong lesion reductions in both groups treated with evernimicin as compared to the untreated, challenged group 2. The group treated with the marketed product bacitracin also showed reduced lesions.

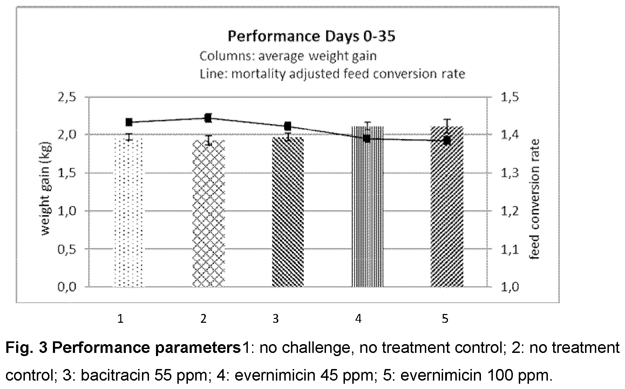

The performance parameters average weight gain (columns) and mortality-adjusted feed conversion rate (line) are depicted in Fig. 3.

Birds treated with evernimicin (groups 4, 5) gained more weight than the birds of the challenged and untreated control (group 2) and surprisingly, gained more weight than the birds treated with the marketed product bacitracin (group 3) and the birds of the unchallenged group (group 1).

The average mortality adjusted feed conversion rate of evernimicin-treated birds was distinctly improved over birds receiving bacitracin or birds receiving no medication (Fig. 3).

### Example 3: In vivo activity of evernimicin in drinking water

The ability of evernimicin to treat and control an outbreak of necrotic enteritis in broiler chicken is demonstrated in an experimental infection model in which medication is administered to the broiler chickens via the drinking water after the infection of birds with a highly pathogenic strain of C. *perfringens.*

In short, immediately after hatching male broiler chicken are divided into 5 groups of 600 birds each. The birds of each group are distributed to 12 pens of 50 birds each. All birds receive starter feed from days 0-13, grower feed from day 13-28 and finisher feed from day 28-35. The study consists of two control groups, two evernimicin-treated groups and one reference group receiving bacitracin (BMD^{®}50), a marketed product for the control and treatment of necrotic enteritis. The groups are depicted in Tab. 4. The first control group does not receive medication nor challenge, whilst the second control group does not receive medication and was challenged on day 17 with a virulent C. *perfringens* chicken isolate. Reference group 3 receives drinking water containing bacitracin at 53 ppm from day 19 to day 25 and 26 ppm until day 35, the label concentration for the control of necrotic enteritis. Evernimicin-treated group 4 receives drinking water containing evernimicin at 53 ppm from day 19 to day 25 and 26 ppm until day 35. Evernimicin-treated group 5 receives drinking water containing evernimicin at 106 ppm from day 19 to day 25 and 26 ppm until day 35.

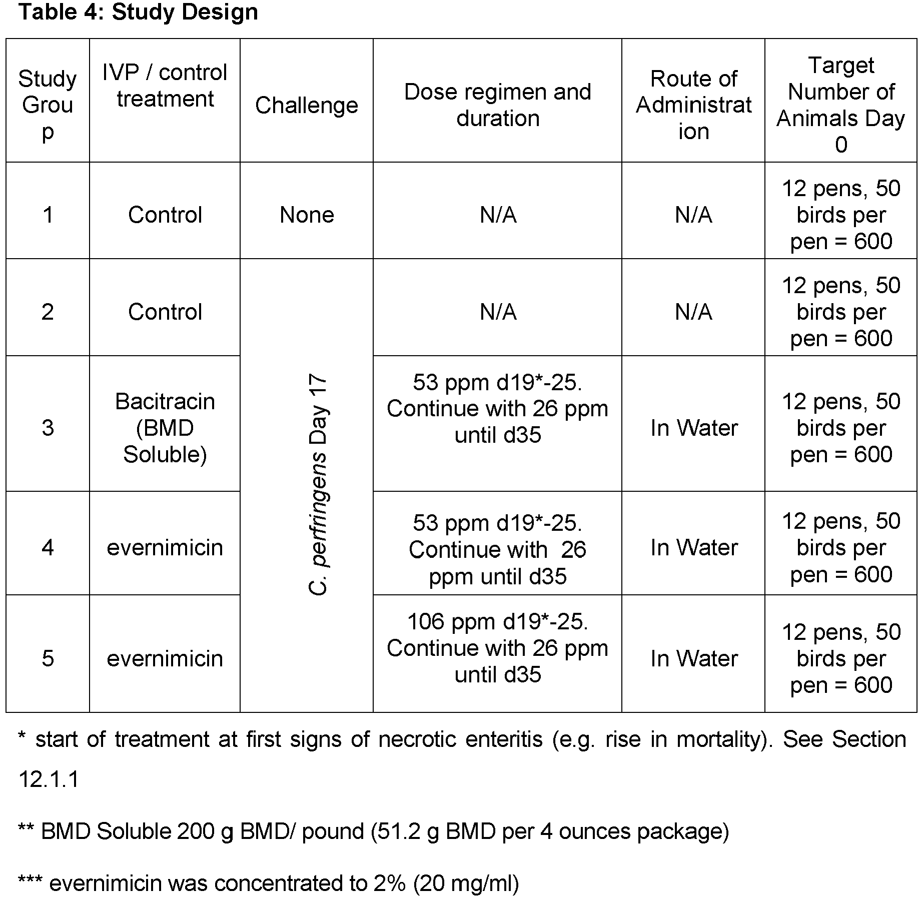

On day 17 the birds in groups 2-5 receive feed containing a virulent C. *perfringens* strain derived from an outbreak of necrotic enteritis in chicken. On Day 19 (±1 day) of study, when bird mortalities from at least 6 separate challenged pens on study are diagnosed as having died with lesions of NE, pens assigned to treatment groups 3, 4 and 5 are issued the assigned medicated treatment water. Pens assigned to treatment groups 1 and 2 are issued fresh water daily (a measured amount of water).

On Day 26 (±1 day) of study, pens assigned to treatment groups 3, 4 and 5 are issued the assigned maintenance medicated treatment water. Pens assigned to treatment groups 1 and 2 are issued fresh water (a measured amount of water).

The primary variables for determining the efficacy of the treatment are:
Percentage of mortality due to NE (caused by C. perfringens). An animal will be considered a study mortality caused by C. perfringens if death/euthanasia occurs following initiation of challenge on Day 17 and the animal has a gross necropsy diagnosis of necrotic enteritis and the animal has a necrotic enteritis lesion score of ≥ 2.

Incidence and mean NE lesion score in sacrificed birds on Day 21 (±1) and Day 23 (±1).

Body weight of surviving birds at Day 36.

Average daily weight gain, feed intake and feed conversion rate from Day 0 to Day 13, Day 13 to 28, Day 28 to 36, Day 17 to Day 36, and Day 0 to Day 36 will be considered performance variables.

### Calculations of Variables

NE percent mortality will be calculated as: (# birds within a pen dying of NE/pen bird count on Day 17) * 100
Incidence of NE lesions will be calculated as: Number of birds within a pen diagnosed with NE lesions/ number of birds within a pen sacrificed
Average daily weight gain (g/d) will be calculated as: [(Pen Weight of all birds remaining at end of period + pen weight of all birds removed during the period)- Pen weight at start of period] / number of bird days in the period for the pen
Average daily feed intake (g/d) will be calculated as: [(Feed remaining in pen at start of period + pen total feed addition weight) - pen feed weigh back weight at end of period] / number of bird days in the period for the pen
Number of bird days on a pen basis = sum of live birds for each day (which includes all remaining live birds at the end of the day plus all dead or culled birds removed during that day) for all days in the period. The calculation will be made from the bird count on body weight days and the dates of removal recorded on the Mortality and Necropsy Record form.

## Claims

1. Evernimicin of the formula 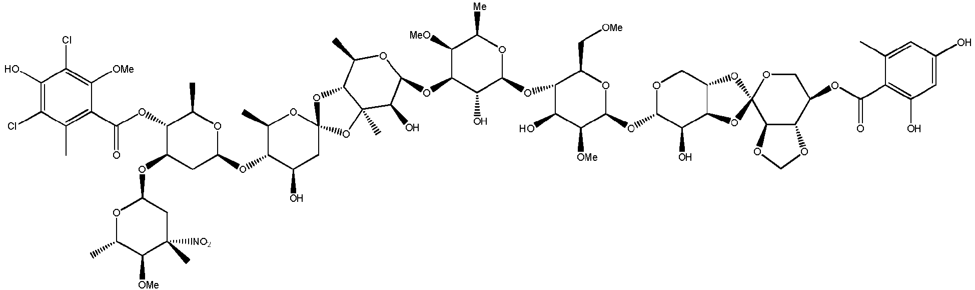 for use in the treatment or prevention of necrotic enteritis in poultry, wherein evernimicin is administered orally.

2. Evernimicin for use according claim 1, wherein the mortality in a treated flock is reduced by 5 to 100%.

3. Evernimicin for use according to any one of claims 1 to 2, wherein evernimicin is used in form of a composition comprising an excipient.

4. Evernimicin for use according to claim 3, wherein the excipient is a pH modifier, preferably adapting the pH value within the range of pH 6.5 to pH 9.

5. Evernimicin for use according to claim 3 or 4, wherein the composition is administered as an aqueous solution.

6. Evernimicin for use according to any one of claims 3 to 5, wherein the composition is administered as an aqueous solution and the concentration of evernimicin is 1 to 500 mg/L, preferably 10 to 200 mg/L, more preferably 40 to 100 mg/L.

7. Evernimicin for use according to any one of claims 1 to 3, wherein evernimicin is administered in-feed containing 1 to 150 ppm, preferably 1 to 100 ppm, more preferably 1 to 45 ppm of evernimicin.

8. Evernimicin for use according to any one of claims 1 to 7, wherein the composition is administered for a duration of 1 to 49 days, preferably for 7 to 21 days.

9. Evernimicin for use according to any one of claims 3 to 6 and 8, wherein the composition is administered orally as an aqueous solution at a concentration of evernimicin of 1 to 500 mg/L for 1 to 14 days for the treatment of necrotic enteritis.

10. Evernimicin for use according to any one of claims 1 to 4, wherein evernimicin is administered orally in feed containing 30 to 150 ppm of evernimicin for 1 to 49 days for the prevention of necrotic enteritis.

11. Evernimicin for use according to any one of claims 1 to 10, wherein the composition is ordered as an aqueous solution at a concentration of evernimicin of 1 to 500 mg/L and/or evernimicin is administered in feed containing 1 to 150 ppm of evernimicin.

12. Kit of parts comprising evernimicin for use according to any one of claims 1 to 11 and instructions of administration.

## Patentansprüche

1. Evernimicin der Formel 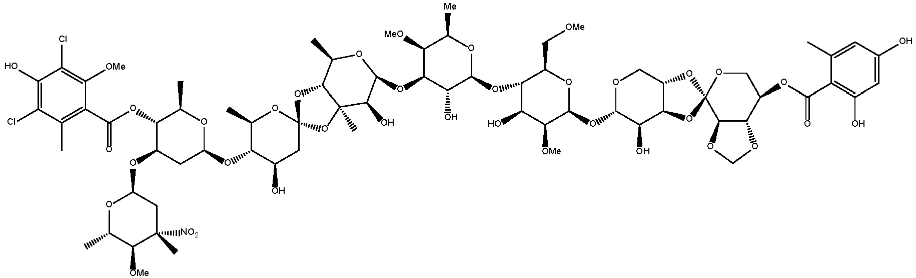 zur Behandlung oder Prävention von nekrotisierender Enteritis bei Geflügel, wobei Evernimicin oral verabreicht wird.

2. Evernimicin zur Verwendung gemäß Anspruch 1, wobei die Sterblichkeit in einer behandelten Herde um 5 bis 100 % reduziert wird.

3. Evernimicin zur Verwendung gemäß einem der Ansprüche 1 bis 2, wobei Evernimicin in Form einer Zusammensetzung verwendet wird, die einen Hilfsstoff umfasst.

4. Evernimicin zur Verwendung gemäß Anspruch 3, wobei der Hilfsstoff ein pH-Modifizierer ist, vorzugsweise zur Anpassung des pH-Werts im Bereich von pH 6,5 bis pH 9.

5. Evernimicin zur Verwendung gemäß Anspruch 3 oder 4, wobei die Zusammensetzung als wässrige Lösung verabreicht wird.

6. Evernimicin zur Verwendung gemäß einem der Ansprüche 3 bis 5, wobei die Zusammensetzung als wässrige Lösung verabreicht wird und die Konzentration von Evernimicin 1 bis 500 mg/L beträgt, vorzugsweise 10 bis 200 mg/L, noch bevorzugter 40 bis 100 mg/L.

7. Evernimicin zur Verwendung gemäß einem der Ansprüche 1 bis 3, wobei Evernimicin im Futter mit 1 bis 150 ppm, vorzugsweise 1 bis 100 ppm, noch bevorzugter 1 bis 45 ppm Evernimicin verabreicht wird.

8. Evernimicin zur Verwendung gemäß einem der Ansprüche 1 bis 7, wobei die Zusammensetzung über einen Zeitraum von 1 bis 49 Tagen, vorzugsweise für 7 bis 21 Tage, verabreicht wird.

9. Evernimicin zur Verwendung gemäß einem der Ansprüche 3 bis 6 und 8, wobei die Zusammensetzung oral als wässrige Lösung mit einer Konzentration von Evernimicin von 1 bis 500 mg/L für 1 bis 14 Tage zur Behandlung von nekrotisierender Enteritis verabreicht wird.

10. Evernimicin zur Verwendung gemäß einem der Ansprüche 1 bis 4, wobei Evernimicin oral in Futter mit 30 bis 150 ppm Evernimicin für 1 bis 49 Tage zur Prävention von nekrotisierender Enteritis verabreicht wird.

11. Evernimicin zur Verwendung gemäß einem der Ansprüche 1 bis 10, wobei die Zusammensetzung als wässrige Lösung mit einer Konzentration von Evernimicin von 1 bis 500 mg/L verabreicht wird und/oder Evernimicin im Futter mit 1 bis 150 ppm Evernimicin verabreicht wird.

12. Kit der Evernimicin zur Verwendung gemäß einem der Ansprüche 1 bis 11 umfasst und Anweisungen zur Verabreichung.

## Revendications

1. Evernimicine de la formule 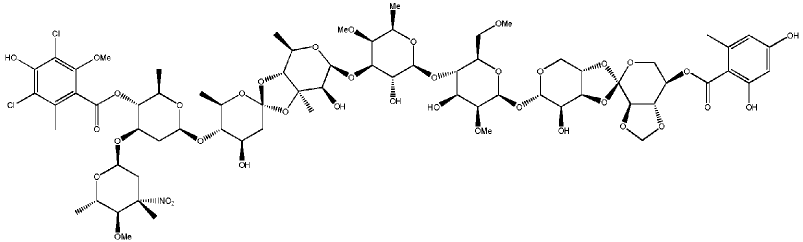 pour une utilisation dans le traitement ou la prévention de l'entérite nécrotique chez la volaille, dans laquelle l'évernimicine est administrée par voie orale.

2. Evernimicine pour une utilisation selon la revendication 1, dans laquelle la mortalité dans un troupeau traité est réduite de 5 à 100 %.

3. Evernimicine pour une utilisation selon l'une quelconque des revendications 1 à 2, dans laquelle l'évernimicine est utilisée sous la forme d'une composition comprenant un excipient.

4. Evernimicine pour une utilisation selon la revendication 3, dans laquelle l'excipient est un modificateur de pH, adaptant de préférence la valeur de pH dans la plage de pH 6,5 à pH 9.

5. Evernimicine pour une utilisation selon la revendication 3 ou 4, dans laquelle la composition est administrée sous forme d'une solution aqueuse.

6. Evernimicine pour une utilisation selon l'une quelconque des revendications 3 à 5, dans laquelle la composition est administrée sous forme d'une solution aqueuse et la concentration d'évernimicine est de 1 à 500 mg/L, préférablement de 10 à 200 mg/L, plus préférablement de 40 à 100 mg/L.

7. Evernimicine pour une utilisation selon l'une quelconque des revendications 1 à 3, dans laquelle l'évernimicine est administrée dans un aliment pour animaux contenant 1 à 150 ppm, préférablement 1 à 100 ppm, plus préférablement 1 à 45 ppm d'évernimicine.

8. Evernimicine pour une utilisation selon l'une quelconque des revendications 1 à 7, dans laquelle la composition est administrée pendant une durée de 1 à 49 jours, préférablement pendant 7 à 21 jours.

9. Evernimicine pour une utilisation selon l'une quelconque des revendications 3 à 6 et 8, dans laquelle la composition est administrée par voie orale sous forme d'une solution aqueuse à une concentration d'évernimicine de 1 à 500 mg/L pendant 1 à 14 jours pour le traitement de l'entérite nécrotique.

10. Evernimicine pour une utilisation selon l'une quelconque des revendications 1 à 4, dans laquelle l'évernimicine est administrée par voie orale dans un aliment pour animaux contenant 30 à 150 ppm d'évernimicine pendant 1 à 49 jours pour la prévention de l'entérite nécrotique.

11. Evernimicine pour une utilisation selon l'une quelconque des revendications 1 à 10, dans laquelle la composition est commandée sous forme d'une solution aqueuse à une concentration d'évernimicine de 1 à 500 mg/L et/ou l'évernimicine est administrée dans un aliment pour animaux contenant 1 à 150 ppm d'évernimicine.

12. Kit de pièces comprenant de l'évernimicine pour une utilisation selon l'une quelconque des revendications 1 à 11 et des instructions d'administration.
